# FASCICULE DE BREVET EUROPEEN

(11) **EP 3 334 387 B1**
(45) Date de publication et mention de la délivrance du brevet: **30.12.2020**
(21) Numéro de dépôt: 16770038.4
(22) Date de dépôt: 11.08.2016
(51) Int. Cl.: A61F 2/44

(54) **DISPOSITIF DE MAINTIEN D'UN ESPACE INTERVERTÉBRAL**
VORRICHTUNG ZUR BEWAHRUNG EINES ZWISCHENWIRBELRAUMS
DEVICE FOR MAINTAINING AN INTERVERTEBRAL SPACE

(30) Priorité: 11.08.2015 FR 1557659; 17.12.2015 FR 1562563
(43) Date de publication de la demande: 20.06.2018
(73) Titulaire: SC Medica, 67000 Strasbourg (FR)
(72) Inventeur: SROUR, Robin, 67200 Strasbourg (FR); SROUR, Camille, 67000 Strasbourg (FR)
(74) Mandataire: Cabinet Bleger-Rhein-Poupon
(86) Numéro de dépôt international: PCT/FR2016/052072
(87) Numéro de publication internationale: WO 2017/025694

(56) Documents cités:
- EP-A1- 2 058 014
- WO-A1-2009/143496
- WO-A1-2010/147829
- US-A- 5 059 193
- US-A1- 2004 249 459
- US-A1- 2010 217 335

## Description

L'invention concerne un dispositif de maintien d'un espace intervertébral destiné à être inséré à l'intérieur d'un disque intervertébral situé entre deux vertèbres voisines, notamment lombaires ou cervicales, en vue de rétablir et maintenir l'espace intervertébral après une discectomie, consécutive à une discopathie avec ou sans hernie discale.

Un disque intervertébral est un fibro cartilage en forme de lentilles biconvexes interposé entre les vertèbres. Il est constitué d'une enveloppe fibreuse et d'un noyau gélatineux enfermé dans l'enveloppe fibreuse.

On parle de discopathie en cas de dégénérescence du disque due à l'âge, aux facteurs génétiques, aux contraintes imposées à la colonne vertébrale, etc.

On parle de hernie discale lorsque, suite à un écrasement du disque, une partie du noyau gélatineux déchire l'enveloppe fibreuse et sort de l'enveloppe fibreuse. La douleur vient principalement du fait que le noyau entre en contact avec les nerfs adjacents.

Le traitement de la hernie discale se fait classiquement de la manière suivante. Après incision de la peau du patient, le chirurgien découpe par chirurgie la partie du noyau gélatineux en protrusion (« discectomie »), c'est-à-dire la partie qui sort du disque. Il retire également ce qu'il peut du reste du noyau dans le disque, pour éviter qu'il ne ressorte par la déchirure de l'enveloppe fibreuse. Cependant, une partie du noyau reste toujours inaccessible et constitue une source potentielle de hernie à venir.

Ensuite, il referme l'incision dans la peau du patient.

Le rétablissement de l'espace intervertébral n'est pas assuré. Au contraire, le retrait du noyau entraine généralement une diminution de l'espace intervertébral.

Alternativement, lorsque le disque est trop abîmé, une opération beaucoup plus lourde doit être envisagée : l'ablation quasi complète du disque et son remplacement par un implant intersomatique ou cage intersomatique (par exemple, technique PLIF pour « Posterior Lumbar Interbody Fusion » ou « fusion intersomatique lombaire postérieure », ou technique TUF pour « Transforaminal Lumbar Interbody Fusion» ou «fusion intersomatique lombaire transforaminale »). Ces cages intersomatiques sont bien connues dans l'art antérieur, par exemple du document WO2005055869.

Elles comportent, en général, une première partie constituée de quatre parois verticales de forme idoine, délimitant entre elles un volume ouvert conçu pour recevoir un produit de comblement osseux, de type greffon ou substitut osseux, destiné à venir en contact avec l'os spongieux des plateaux vertébraux pour favoriser la fusion osseuse entre les deux vertèbres. Ces cages intersomatiques sont généralement très stables, mais le corollaire de cette stabilité est qu'elles présentent une grande résistance aux forces de traction et de compression que lui imprime la colonne vertébrale. Finalement, elles provoquent une fusion des vertèbres adjacentes, qui n'ont plus de mobilité intervertébrale.

Le document WO2010/147829 décrit un implant intervertébral expansible entre une position sensiblement tubulaire, vers une position sensiblement sphérique.

Ce type d'implant expansible ne permet pas de résister aux pressions intervertébrales qui sont très élevées, notamment au niveau des vertèbres lombaires. Ceci est précisément dû à leur caractère expansible. En effet, ces dispositifs doivent répondre à des exigences contradictoires et incompatibles, à savoir être suffisamment souple pour pouvoir être mis dans leur forme finale sphérique manuellement par le médecin, et suffisamment rigide pour résister aux forces en compression intervertébrales, de l'ordre de 700 à 800 daN au niveau lombaire.

En outre, ce type d'implant n'ayant pas une rigidité importante nécessite une insertion dans l'espace intervertébral via une vertèbre à perforer, comme le montre le document WO2009/143496. Cette insertion par voie mini-invasive/percutanée est très traumatisante, destructrice de la vertèbre voisine qui doit se réparer progressivement. Cette technique implique une formation additionnelle et des nouvelles compétences à acquérir pour la grande majorité des praticiens concernés.

La présente invention permet de traiter les discopathies, avec ou sans hernie discale, par une voie d'insertion très peu traumatisante et totalement impossible à suivre avec un dispositif expansible.

L'invention vise principalement à permettre un traitement complémentaire des hernies discales sans ablation du disque intervertébral permettant un rétablissement et un maintien de l'espace intervertébral, une diminution des risques de récidive (nouvelle éjection du reste du noyau gélatineux), ainsi que le maintien de la liberté de mouvement de la colonne (flexion vers l'avant, extension vers l'arrière, inclinaison latérale, et rotation). Elle permet également dans certaines indications chirurgicales de remplacer les cages intersomatiques.

L'objectif de l'invention est donc de proposer un dispositif capable de se loger à l'intérieur du disque, à la place du noyau, d'assurer son ancrage naturel (sans intervention manuelle du chirurgien qui ne peut accéder à l'intérieur du disque) en favorisant la repousse fibreuse autour d'au moins une partie du dispositif, de limiter la possibilité d'éjection du noyau résiduel pour éviter une nouvelle hernie, et d'assurer la mobilité de la colonne vertébrale.

Le dispositif selon l'invention doit permettre de résister aux forces en compression axiale statique des tests médicaux modernes (marquage CE par exemple) d'au moins 2000 daN, de préférence d'au moins 3000 daN, tout en permettant un protocole d'insertion simple et non traumatisant.

À cette fin, l'invention a pour objet un dispositif de maintien d'un espace intervertébral destiné à être disposé à l'intérieur d'un disque intervertébral caractérisé en ce qu'il comprend au moins deux anneaux concentriques non expansibles ménageant, entre eux, un espace libre, et reliés l'un à l'autre par deux sommets de jonction.

Par non expansibles, on comprend que le dispositif ne possède qu'une seule conformation, sa conformation d'utilisation, qui lui confère une grande rigidité mécanique et une grande solidité, sans risque d'affaissement ou de rupture entre deux parties mobiles l'une par rapport à l'autre, comme dans les implants expansibles.

Selon des modes de réalisation particuliers :
- les anneaux peuvent être elliptiques et présenter un rayon maximal et un rayon minimal, le rapport entre le rayon maximal et le rayon minimal étant compris entre 1 :1 et 1,5 :1 ;
- les anneaux peuvent être de rayon maximal identiques et de rayon minimal identiques ;
- un pilier de renfort peut être positionné dans un anneau ;
- un pilier de renfort peut être positionné dans chaque anneau, de préférence de manière symétrique par rapport à un centre du dispositif de maintien ;
- un pilier de renfort peut être positionné entre deux anneaux ;
- deux piliers de renfort peuvent être positionnés chacun entre deux anneaux, de préférence de manière symétrique par rapport à un centre du dispositif de maintien ;
- un orifice taraudé peut être agencé au centre d'un sommet de jonction ;
- le dispositif peut comprendre, en outre, un orifice non taraudé agencé dans au moins un anneau, l'orifice taraudé et l'orifice non taraudé présentant des axes de révolution parallèles l'un par rapport à l'autre.

L'invention a également pour objet un procédé de fabrication d'un dispositif de maintien d'un espace intervertébral précédent, consistant à agréger couche par couche des particules biocompatibles, de préférence de titane.

En particulier, le procédé est mis en œuvre avec une imprimante tridimensionnelle.

D'autres caractéristiques de l'invention seront énoncées dans la description détaillée ci-après, faite en référence aux dessins annexés, qui représentent, respectivement :
- la figure 1, une vue schématique en coupe d'une portion de colonne vertébrale présentant une hernie discale ;
- la figure 2, une vue schématique en coupe de la portion de colonne vertébrale de la figure 1 après ablation d'une partie du noyau ;
- la figure 3, une vue schématique en coupe de la portion de colonne vertébrale de la figure 2 dans laquelle un dispositif de maintien d'un espace intervertébral selon l'invention est en cours de positionnement;
- la figure 4, une vue schématique en coupe de la portion de colonne vertébrale de la figure 3 dans laquelle un dispositif de maintien d'un espace intervertébral selon l'invention est en position d'utilisation ;
- la figure 5, une vue schématique en perspective d'un premier mode de réalisation d'un dispositif de maintien d'un espace intervertébral selon l'invention ;
- la figure 6, une vue schématique en plan du mode de réalisation de la figure 5, vue face à l'orifice taraudé ;
- la figure 7, une vue schématique en plan d'un deuxième mode de réalisation d'un dispositif de maintien d'un espace intervertébral selon l'invention ;
- la figure 8, une vue schématique en plan quotté d'un exemple de réalisation d'un dispositif de maintien d'un espace intervertébral selon l'invention ;
- la figure 9, une vue schématique en perspective d'un troisième mode de réalisation d'un dispositif de maintien d'un espace intervertébral selon l'invention équipé de renforts intra-annulaires ;
- la figure 10, une vue schématique en coupe d'un quatrième mode de réalisation d'un dispositif de maintien d'un espace intervertébral selon l'invention équipé de renforts inter-annulaires ;
- la figure 11, une vue schématique en coupe d'un outil de positionnement d'un dispositif de maintien d'un espace intervertébral selon l'invention ;
- la figure 12, une vue schématique en plan de côté d'un mode de réalisation alternatif d'un outil de positionnement d'un dispositif de maintien d'un espace intervertébral selon l'invention ;
- la figure 13, une vue schématique en perspective de l'extrémité de fixation de l'outil de positionnement de la figure 12 ; et
- la figure 14, une vue schématique en perspective de l'extrémité de fixation de l'outil de positionnement de la figure 13 sur laquelle est vissé un dispositif de maintien d'un espace intervertébral selon l'invention ;
- la figure 15, une vue schématique en perspective d'un cinquième mode de réalisation d'un dispositif de maintien d'un espace intervertébral selon l'invention ;
- la figure 16, une vue schématique en coupe d'un sixième mode de réalisation d'un dispositif de maintien d'un espace intervertébral selon l'invention équipé d'un renfort annulaire ;
- la figure 17, une vue schématique en perspective d'un septième mode de réalisation d'un dispositif de maintien d'un espace intervertébral selon l'invention équipé de renforts inter-annulaires multiples ; et
- la figure 18, une vue schématique en perspective d'un huitième mode de réalisation d'un dispositif de maintien d'un espace intervertébral selon l'invention équipé de renforts inter-annulaires grillagés.

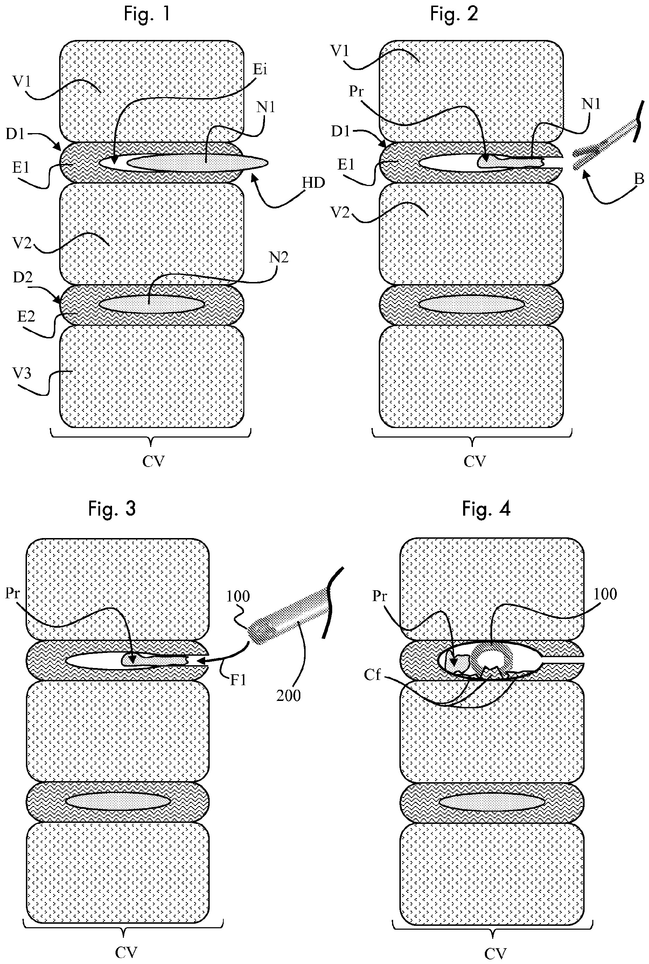

Les figures 1 et 2 illustrent les étapes classiques de chirurgie d'une hernie discale ou discectomie. Ces mêmes étapes et l'invention peuvent être également utilisées pour une discopathie sans hernie discale associée.

Lors de l'opération, le chirurgien incise la peau du patient afin d'accéder au disque D1 situé entre les vertèbres V1 et V2 de la portion de colonne vertébrale CV (figure 1). Ce disque D1 comprend une enveloppe fibreuse E1 et un noyau gélatineux N1 qui a déchiré l'enveloppe E1 et se trouve en protrusion, formant la hernie discale HD.

Par comparaison, le disque D2 situé entre les vertèbres V2 et V3 présente un aspect normal, où le noyau N2 est confiné dans l'espace intra discal Ei, au centre de l'enveloppe E2.

Puis à l'aide d'un outil de découpe chirurgicale B (figure 2), de type bistouri et pince à disque, le chirurgien découpe la partie du noyau dépassant du disque, et découpe ce qu'il peut du reste du noyau à l'intérieure de l'ouverture dans le disque (discectomie). Il reste alors une portion résiduelle de noyau Pr.

Cette technique classique de discectomie de hernie discale, dite "à ciel ouvert" est l'opération du rachis la plus pratiquée (22% des 152 372 opérations du rachis réalisées en France en 2013 selon l'Agence technique de l'information sur l'hospitalisation).

Grâce à l'invention, le chirurgien peut rétablir et maintenir l'espace intervertébral durant cette opération classique, directement après l'ablation de la hernie discale, sans nécessiter de perforer une vertèbre voisine, ni retourner le patient.

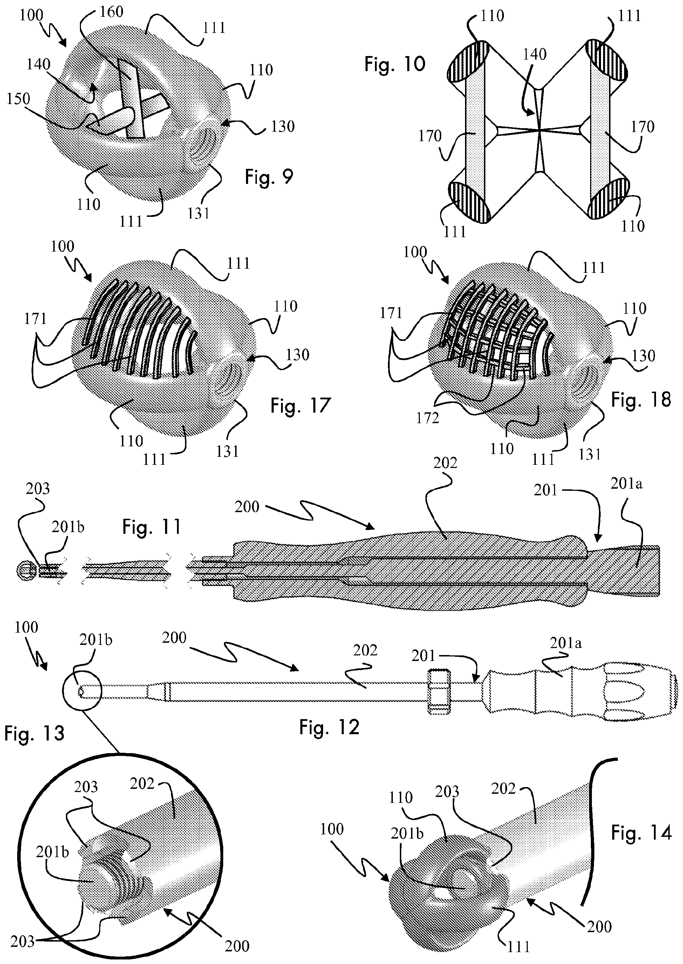

Pour cela, le chirurgien insère en force, directement dans l'espace intervertébral via l'orifice crée par la hernie discale, (figure 3) un dispositif 100 de maintien d'un espace intervertébral selon l'invention, en donnant des coups de marteau sur l'extrémité extérieure d'un outil porte-implant 200, qui sera décrit plus en détail en rapport aux figures 11 à 14.

Le médecin aura préalablement placé et retiré des implants d'essai (dits « implants fantômes ») de diamètres différents pour préparer la niche et déterminer la taille adaptée au patient du dispositif de maintien d'un espace intervertébral selon l'invention.

L'insertion selon la flèche F1 se fait dans la partie déchirée de l'enveloppe du disque, ce qui repousse la portion résiduelle de noyau Pr vers l'intérieur de l'espace intra discal (figure 4).

Comme il sera expliqué plus en détail par la suite, le dispositif 100 selon l'invention permet également une insertion facile grâce à sa forme extérieure elliptique, de préférence sphérique. En outre, l'utilisation d'anneaux pour constituer le dispositif 100 permet de libérer un passage important pour les tissus de l'enveloppe, participant à la facilité d'insertion, et permet une colonisation des cellules fibreuses Cf, participant à l'ancrage naturel du dispositif selon l'invention.

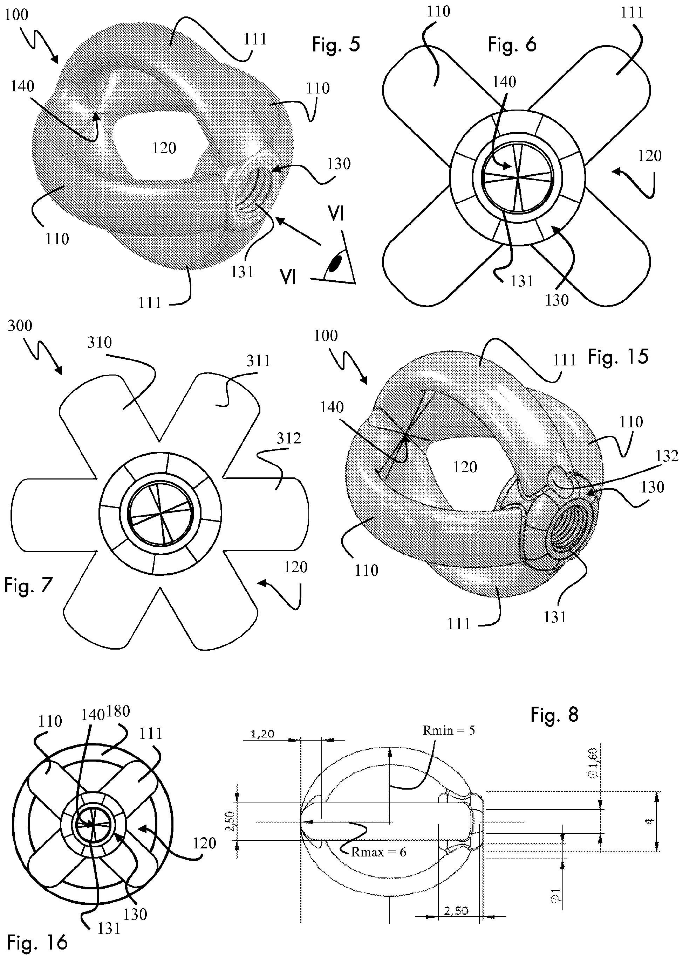

Un premier mode de réalisation d'un dispositif 100 de maintien d'un espace intervertébral selon l'invention est illustré en figure 5.

Le dispositif 100 comprend, dans cet exemple, deux anneaux 110 et 1 1 1 non expansibles, concentriques et qui sont de rayons Rmin identiques et de rayons Rmax identiques. Autrement dit, les anneaux sont identiques et concentriques. Ils sont reliés l'un à l'autre par deux sommets de jonction 130 et 140, ménageant, entre eux, un espace libre 120.

Dans le mode de réalisation illustré en figure 8, les anneaux sont elliptiques et présentent un rayon maximal Rmax et un rayon minimal Rmin, le rapport entre le rayon maximal Rmax et le rayon minimal Rmin étant compris entre 1 :1 et 1,5 :1.

Dans le mode de réalisation illustré en figure 5, les anneaux sont circulaires et présentent un rayon maximal Rmax et un rayon minimal Rmin identiques, le rapport entre le rayon maximal Rmax et le rayon minimal Rmin étant égal à 1:1.

À titre indicatif et non limitatif, dans l'application concernée, Rmin est compris entre 2 et 7 mm, les plus petites tailles étant généralement réservées pour les vertèbres cervicales et les plus grandes tailles pour les vertèbres lombaires.

Ils présentent une section telle que la surface de contact entre les anneaux et les tissus du patient est arrondie vers l'extérieure du dispositif selon l'invention.

Un orifice taraudé 131 est agencé au centre de l'un sommet de jonction 130. Il permet la fixation à l'outil de positionnement 200 (voir figures 11 à 14) qui comporte une molette 201 munie d'une extrémité de préhension 201a et d'une extrémité filetée 201b destinée à être vissée dans l'orifice taraudé 131, la molette 201 étant montée rotative dans un manche 202.

Afin de permettre le dévissage de l'outil de positionnement 200 sans faire tourner le dispositif de maintien d'un espace intervertébral selon l'invention, le manche 202 de l'outil de positionnement comporte au moins deux tétons 203, de préférence quatre tétons 203 (voir figure 13), destinés à et conformés pour s'insérer entre deux anneaux 110-111 (voir figure 14).

Alternativement, il peut être prévu au moins un orifice non taraudé agencé dans au moins un des anneaux 110-111, et destiné à recevoir un téton de blocage 203 porté par l'outil de positionnement. L'orifice taraudé 131 et le ou les orifices non taraudés 132 présentent des axes de révolution parallèles les uns par rapport aux autres.

Sur la figure 15, il est prévu deux orifices non taraudés 132 agencés dans l'anneau 111, de part et d'autre de l'orifice taraudé 131, et destinés à recevoir deux tétons de blocage 203 portés par l'outil de positionnement.

La molette filetée permet de fixer le dispositif selon l'invention sur l'outil de positionnement 200, et les tétons 203 permettent de maintenir le dispositif en position et éviter qu'il ne tourne avec la molette. Il est ainsi possible de faire entrer le dispositif à l'intérieur du disque tout en appliquant des petits mouvements oscillant ou en tapant dessus avec un marteau, sans risque de dévisser le dispositif de l'outil de positionnement. Les tétons 203 empêchent le dévissage.

Lorsque le chirurgien considère que le dispositif est en bonne position, il dévisse la molette 201. Le dispositif 100 n'est plus fixé au manche que par les tétons 203 (bloqués entre deux anneaux ou dans les orifices non taraudés 132), de sorte que le chirurgien n'a qu'à retirer délicatement l'outil 200, laissant le dispositif 100 selon l'invention en position d'utilisation, à l'intérieur du disque intervertébral.

La structure en anneaux concentriques présente de nombreux avantages. Tout d'abord, elle permet de libérer un espace 120 entre les anneaux qui facilite l'introduction du dispositif en permettant aux tissus de se déformer vers l'intérieur du dispositif.

En outre, cet espace 120 entre les anneaux 110-111 permet une colonisation rapide par les tissus fibreux venant de l'enveloppe. Le dispositif peut donc s'ancrer naturellement et relativement rapidement. Alternativement, ou en combinaison, il peut être prévu d'injecter un produit d'ancrage (de type colle, ciment ou greffon osseux) ou de favorisation du développement cellulaire au travers de l'orifice 130. Tout autre produit qui pourrait être nécessaire à l'opération peut également être injecté.

Dans ce cas, il est avantageusement prévu, selon l'invention, que la molette filetée 201 de l'outil de positionnement 200 comporte un canal central relié à un réservoir de produit (non illustré). Le produit peut alors être injecté avant de dévisser la molette 201.

La structure en anneaux concentriques non expansibles permet également de repousser le noyau résiduel grâce au sommet de jonction 140 opposé au sommet de jonction 130.

Le dispositif selon l'invention permet ainsi de limiter les risques de sortie du noyau résiduel en le repoussant et en maintenant une pression intra discale suffisamment faible pour éviter l'expulsion du noyau résiduel. Il permet de maintenir la hauteur discale, de diminuer la pression sur le disque résiduel et d'éviter ainsi la récidive herniaire. Il permet également de préserver les étages vertébraux adjacents.

La structure en anneaux concentriques non expansibles permet également d'assurer une mobilité optimale de la colonne vertébrale qui peut toujours pivoter grâce au dispositif elliptique 100 (de préférence sphérique) selon l'invention tout en bénéficiant des propriétés mécaniques naturelles de l'enveloppe du disque qui reste en place. À cette fin, le rapport entre le rayon maximal Rmax et le rayon minimal Rmin est inférieur à 1,5 :1, de préférence égal à 1 :1. Autrement dit, il est possible de fabriquer un dispositif selon l'invention légèrement ovoïdal, par exemple pour augmenter la surface de contact avec le corps du patient, mais il est nécessaire de limiter la longueur à une fois et demie la hauteur.

Avec un dispositif expansible, il serait impossible d'utiliser la voie d'insertion selon l'invention : on insérerait puis on ouvrirait le dispositif. Cette ouverture serait impossible manuellement sans soulager les vertèbres. Pour contrer la pression intervertébrale, il serait donc nécessaire d'utiliser des écarteurs, ce qui augmente de manière rédhibitoire le risque de traumatisme additionnel et de complications opératoires.

Grâce à la structure non expansible du dispositif selon l'invention, l'insertion en force est possible. La forme sphérique/elliptique évite tout risque de traumatisme lors de cette insertion, tout en permettant d'écarter les vertèbres juste suffisamment pour placer correctement l'implant. Il n'y a donc pas de risque de sur-écarter les vertèbres.

Enfin, la structure en anneaux concentriques permet d'obtenir une bonne stabilité du dispositif grâce à la repousse fibreuse qui va coloniser ce dispositif, tout en limitant les zones de contact avec les tissus du patient grâce aux sommets arrondis des anneaux de l'invention. Les risques d'abîmer ces tissus sont donc limités.

Dans un mode de réalisation alternatif, illustré en figure 7, le dispositif 300 selon l'invention comporte plus de deux anneaux : il comporte trois anneaux concentriques 310, 311 et 312. Les autres caractéristiques restent identiques.

L'augmentation du nombre d'anneaux peut permettre un meilleur contrôle lors du positionnement du dispositif et un meilleur contrôle de la mobilité de la colonne après opération.

Néanmoins, en augmentant le nombre d'anneaux, on augmente le coût de l'implant et on limite l'espace entre les anneaux. Il convient, par conséquent, d'adapter le nombre d'anneaux et leur section pour que l'espace entre les anneaux permette une bonne colonisation des cellules fibreuses. En pratique, on s'arrangera de préférence pour que l'espace libre 120 entre les anneaux soit d'au moins 30 mm³, tout en conservant une structure sphérique ou quasi sphérique (lorsque le rapport entre le rayon maximal Rmax et le rayon minimal Rmin est supérieur à 1 :1 et inférieur ou égal à 1,5 :1), Rmin étant compris entre 2 et 7 mm.

Le dispositif selon l'invention est donc compact tout en offrant un espace intérieur important permettant une bonne colonisation des cellules fibreuses. Grâce à se structure non expansible, il peut donc être facilement inséré et s'ancrer naturellement (par la repousse fibreuse) dans le corps.

Le dispositif 100-300 selon l'invention de maintien d'un espace intervertébral est avantageusement obtenu par fabrication additive par couches. Ainsi, il est fabriqué par agrégation couche par couche de particules, de préférence de titane pour sa solidité et sa biocompatibilité.

La section et le matériau des anneaux est choisie pour permettre au dispositif selon l'invention de résister à des forces en compression axiale statique d'au moins 2000 daN, de préférence d'au moins 3000 daN, tout en respectant les dimensions intervertébrales optimales, ce qui est possible grâce au caractère non expansible du dispositif selon l'invention. Par résister, on entend que le dispositif selon l'invention ne de doit pas d'écraser ou rompre lorsqu'il est soumis à une force en compression axiale statique d'au moins 2000 daN, de préférence d'au moins 3000 daN.

Par exemple, un implant selon l'invention est en titane, et il présente un diamètre général compris entre 7,5 et 14 mm. Chaque anneau présente une section transversale sensiblement circulaire ou elliptique entre 1,2 et 2,5 mm de diamètre.

Un dispositif expansible ne pourrait pas être conformer pour une telle résistance, au risque de ne pas pouvoir être déployé.

Le procédé d'agrégation couche par couche est avantageusement mis en oeuvre par une imprimante tridimensionnelle.

Pour s'assurer de la rigidité du dispositif, il peut avantageusement être prévu des renforts internes, situés au sein du dispositif.

La figure 9 illustre un mode de réalisation dans lequel un pilier de renfort 150-160 est positionné dans chaque anneau, de préférence de manière symétrique par rapport à un centre du dispositif de maintien (constitué par le barycentre de la sphère ou de l'ovoïde). En d'autres termes, dans ce mode de réalisation, le dispositif comporte des renforts intra-annulaires, chaque pilier de renfort étant en contact avec deux parties diamétralement opposées d'un même anneau.

La figure 10 illustre un mode de réalisation dans lequel un pilier 170 de renfort est positionné entre deux anneaux 110-111 .

De préférence, au moins deux piliers 170 de renfort sont positionnés entre deux anneaux 110-111 de préférence de manière symétrique par rapport au centre du dispositif de maintien. En d'autres termes, dans ce mode de réalisation, le dispositif comporte des renforts inter-annulaires, chaque pilier de renfort étant en contact avec deux parties de deux anneaux 110-111 différents.

La figure 16 illustre un mode de réalisation particulier de la figure 10, dans lequel un pilier de renfort 180 est positionné perpendiculairement aux anneaux 110-111 . Avantageusement, ce pilier de renfort 180 est constitué par un anneau de renfort positionné perpendiculairement aux anneaux 110-111 .

Bien entendu, un dispositif de maintien de l'espace intervertébral selon l'invention peut comprendre une combinaison des moyens de renforts illustrés aux figures 9, 10 et 16.

Ainsi, par exemple, la figure 17 illustre un mode de réalisation dans lequel le dispositif de maintien de l'espace intervertébral comporte une pluralité de piliers 171 agencés les uns à côté des autres (parallèlement ou radialement) et en contact avec deux moitiés de deux anneaux 110-111 différents. Sur cette figure, seuls un quart du dispositif de maintien de l'espace intervertébral selon l'invention est illustré comme étant pourvu des piliers 171. Bien entendu, en réalité, tous les espaces inter annulaires sont équipés de tels piliers 171 dans ce mode de réalisation.

Cette pluralité de piliers 171 permet de répartir le soutien des anneaux tout en permettant de prévoir des piliers 171 étroits, laissant la place pour la colonisation cellulaire.

Selon un autre mode de réalisation illustré en figure 18, les piliers de renforts 171 sont en outre reliés les uns aux autres par des portions de jonction 172 afin de constituer un grillage de renfort 171-172 positionné entre deux moitiés de deux anneaux 110-111 différents. Sur cette figure, seuls un quart du dispositif de maintien de l'espace intervertébral selon l'invention est illustré comme étant pourvu des piliers 171 et des portions de jonction 172. Bien entendu, en réalité, tous les espaces inter annulaires sont équipés de tels piliers 171 et de telles portions de jonction 172 dans ce mode de réalisation.

Le grillage de renfort 171-172 permet non seulement de rigidifier le dispositif de maintien de l'espace intervertébral selon l'invention, mais il diminue également le risque d'enfoncement du dispositif dans les plateaux vertébraux.

L'espace entre les piliers 171 et les portions de jonction 172 doit néanmoins être maximisé pour assurer une bonne colonisation cellulaire. La maximisation de ces espaces est permise par la grande rigidité des anneaux beaucoup plus épais.

Finalement, le dispositif de maintien de l'espace intervertébral selon l'invention comporte des régions très solides et rigides, que sont les anneaux 110 et 111, et des régions de renfort constituées de piliers 171 et de portions de jonction 172 beaucoup plus fins qui permettent de limiter les risques d'enfoncement dans les plateaux vertébraux. Autrement dit, le dispositif de maintien de l'espace intervertébral selon l'invention est une sphère ou un ovoïde hétérogène, avec des parties non expansibles, épaisses et rigides que sont les anneaux 110-111, et des parties non expansibles plus fines de piliers 171 ou de grillage 171-172 permettant de répartir la charge sur les plateaux vertébraux.

L'invention permet donc un traitement complémentaire des hernies discales, voire des discopathies sans hernies, sans ablation du disque intervertébral permettant un rétablissement et un maintien de l'espace intervertébral, une diminution des risques de récidive (nouvelle éjection du reste du noyau gélatineux), ainsi que le maintien de la liberté de mouvement de la colonne.

Le dispositif selon l'invention se loge facilement à l'intérieur du disque, à la place du noyau, et permet un ancrage naturel en favorisant la repousse fibreuse autour des anneaux.

## Revendications

1. Dispositif (100-300) de maintien d'un espace intervertébral destiné à être disposé à l'intérieur d'un disque intervertébral **caractérisé en ce qu'**il comprend au moins deux anneaux concentriques non expansibles (110-111, 310-31 1-312) ménageant, entre eux, un espace libre (120), et reliés l'un à l'autre par deux sommets de jonction (130-140).

2. Dispositif de maintien d'un espace intervertébral selon la revendication 1, dans lequel les anneaux (110-111, 310-31 1-312) sont elliptiques et présentent un rayon maximal (Rmax) et un rayon minimal (Rmin), le rapport entre le rayon maximal et le rayon minimal étant compris entre 1 :1 et 1,5 :1.

3. Dispositif de maintien d'un espace intervertébral selon la revendication 1 ou 2, dans lequel les anneaux (110-111, 310-31 1-312) sont de rayon maximal (Rmax) identiques et de rayon minimal (Rmin) identiques.

4. Dispositif de maintien d'un espace intervertébral selon l'une quelconque des revendications 1 à 3, dans lequel un pilier de renfort (150-160) est positionné dans un anneau (110-111).

5. Dispositif de maintien d'un espace intervertébral selon l'une quelconque des revendications 1 à 3, dans lequel un pilier de renfort (150-160) est positionné dans chaque anneau (110-111).

6. Dispositif de maintien d'un espace intervertébral selon l'une quelconque des revendications 1 à 3, dans lequel un pilier de renfort (170) est positionné entre deux anneaux (110-111).

7. Dispositif de maintien d'un espace intervertébral selon l'une quelconque des revendications 1 à 3, dans lequel deux piliers de renfort (170) sont positionnés chacun entre deux anneaux (110-111).

8. Dispositif de maintien d'un espace intervertébral selon la revendication 7, dans lequel une pluralité de piliers de renfort (171) sont agencés les uns à côté des autres et en contact avec deux moitiés de deux anneaux différents (110-111).

9. Dispositif de maintien d'un espace intervertébral selon la revendication 8, dans lequel les piliers de renforts (171) sont en outre reliés les uns aux autres par des portions de jonction (172) afin de constituer un grillage de renfort (171-172) positionné entre deux moitiés de deux anneaux différents (110-111).

10. Procédé de fabrication d'un dispositif de maintien d'un espace intervertébral selon l'une quelconque des revendications 1 à 9, consistant à agréger couche par couche des particules biocompatibles, de préférence de titane.

## Patentansprüche

1. Vorrichtung (100-300) zum Aufrechterhalten eines Zwischenwirbelraums, der dazu bestimmt ist, innerhalb einer Wirbelscheibe angeordnet zu werden, **dadurch gekennzeichnet, dass** sie mindestens zwei nicht expandierbare konzentrische Ringe (110-111, 310-311-312) umfasst, die zwischen ihnen einen freien Raum (120) bilden und über zwei Verbindungspunkte (130-140) miteinander verbunden sind.

2. Vorrichtung zum Aufrechterhalten eines Zwischenwirbelraums nach Anspruch 1, bei der die Ringe (110-111, 310-311-312) elliptisch sind und einen maximalen Radius (Rmax) und einen minimalen Radius (Rmin) aufweisen, wobei das Verhältnis zwischen dem maximalen Radius und dem minimalen Radius zwischen 1:1 und 1,5:1 liegt.

3. Vorrichtung zum Aufrechterhalten eines Zwischenwirbelraums nach Anspruch 1 oder 2, bei der die Ringe (110-111, 310-311-312) einen identischen maximalen Radius (Rmax) und einen identischen minimalen Radius (Rmin) haben.

4. Vorrichtung zum Aufrechterhalten eines Zwischenwirbelraums nach irgendeinem der Ansprüche 1 bis 3, bei der eine Verstärkungssäule (150-160) in einem Ring (110-111) positioniert ist.

5. Vorrichtung zum Aufrechterhalten eines Zwischenwirbelraums nach irgendeinem der Ansprüche 1 bis 3, bei der eine Verstärkungssäule (150-160) in jedem Ring (110-111) positioniert ist.

6. Vorrichtung zum Aufrechterhalten eines Zwischenwirbelraums nach irgendeinem der Ansprüche 1 bis 3, bei der eine Verstärkungssäule (170) zwischen zwei Ringen (110-111) positioniert ist.

7. Vorrichtung zum Aufrechterhalten eines Zwischenwirbelraums nach irgendeinem der Ansprüche 1 bis 3, bei der jeweils zwei Verstärkungssäulen (170) zwischen zwei Ringen (110-111) positioniert sind.

8. Vorrichtung zum Aufrechterhalten eines Zwischenwirbelraums nach Anspruch 7, bei der eine Vielzahl von Verstärkungssäulen (171) nebeneinander und in Berührung mit zwei Hälften von zwei unterschiedlichen Ringen (110-111) angeordnet sind.

9. Vorrichtung zum Aufrechterhalten eines Zwischenwirbelraums nach Anspruch 8, bei der die Verstärkungssäulen (171) weiterhin über Verbindungsteile (172) miteinander verbunden sind, um ein Verstärkungsnetz (171-172) zu bilden, das zwischen zwei Hälften von zwei unterschiedlichen Ringen (110-111) positioniert ist.

10. Verfahren zur Herstellung einer Vorrichtung zur Aufrechterhaltung eines Zwischenwirbelraums nach irgendeinem der Ansprüche 1 bis 9, das darin besteht, biokompatible Partikel, vorzugsweise Titanpartikel, schichtweise zu aggregieren.

## Claims

1. A device (100-300) for maintaining an intervertebral space intended to be positioned inside an intervertebral disc, **characterized in that** it comprises at least two non-expandable concentric rings (110-111, 310-311-312) arranging a free space (120) between them, and connected to one another by two junction summits (130-140).

2. The device for maintaining an intervertebral space according to claim 1, wherein the rings (110-111, 310-311-312) are elliptical and have a maximum radius (Rmax) and a minimum radius (Rmin), the ratio between the maximum radius and the minimum radius being comprised between 1:1 and 1.5:1.

3. The device for maintaining an intervertebral space according to claim 1 or 2, wherein the rings (110-111, 310-311-312) have an identical maximum radius (Rmax) and an identical minimum radius (Rmin).

4. The device for maintaining an intervertebral space according to any one of claims 1 to 3, wherein a reinforcing pillar (150-160) is positioned in a ring (110-111).

5. The device for maintaining an intervertebral space according to any one of claims 1 to 3, wherein a reinforcing pillar (150-160) is positioned in each ring (110-111).

6. The device for maintaining an intervertebral space according to any one of claims 1 to 3, wherein a reinforcing pillar (170) is positioned between two rings (110-111).

7. The device for maintaining an intervertebral space according to any one of claims 1 to 3, wherein two reinforcing pillars (170) are positioned each between two rings (110-111).

8. The device for maintaining an intervertebral space according to claim 7, wherein multiple reinforcing pillars (171) are arranged next to one another and in contact with two halves of two different rings (110-111).

9. The device for maintaining an intervertebral space according to claim 8, wherein the reinforcing pillars (171) are further connected to one another by junction portions (172) in order to form a reinforcing mesh (171-172) positioned between two halves of two different rings (110-111).

10. A method for manufacturing a device for maintaining an intervertebral space according to any one of claims 1 to 9, consisting of aggregating biocompatible particles, preferably of titanium, layer by layer.
